# EUROPEAN PATENT APPLICATION

(11) **EP 4 711 755 A1**
(43) Date of publication of application: **18.03.2026**
(21) Application number: 24807122.7
(22) Date of filing: 09.05.2024
(51) Int. Cl.: G01N 27/62

(54) **METHOD FOR ANALYZING DOUBLE-STRANDED OLIGONUCLEOTIDE**

(30) Priority: 12.05.2023 JP 2023079674
(71) Applicant: SHIMADZU CORPORATION, Kyoto-shi, Kyoto 604-8511 (JP)
(72) Inventor: FUKUYAMA, Yuko, Kyoto-shi, Kyoto 604-8511 (JP)
(74) Representative: Müller-Boré & Partner Patentanwälte PartG mbB
(86) International application number: PCT/JP2024/017300
(87) International publication number: WO 2024/237170

(57) **Abstract**

A method for analyzing a double-stranded oligonucleotide included in a sample uses an ion trap mass spectrometer comprising an ion source based on matrix-assisted laser desorption/ionization, an ion trap unit for trapping and ejecting ions generated in the ion source, and a detection unit for detecting the ions ejected from the ion trap unit. The method involves introducing the sample into the ion source, irradiating the sample with a laser, trapping the ions generated in the ion source in the ion trap unit, and then ejecting and detecting the ions trapped in the ion trap unit. A delay time, which is the time from irradiating the sample with the laser in the ion source until applying a trapping voltage to trap ions in the ion trap unit, is set to be longer than a value preset for the ion trap mass spectrometer as a delay time for trapping ions with a mass-to-charge ratio comparable to that corresponding to the molecular weight of the double-stranded oligonucleotide. This allows for the analysis of the double-stranded oligonucleotide.

## Description

### TECHNICAL FIELD

The present invention relates to a method for analyzing a double-stranded oligonucleotide.

### BACKGROUND ART

Oligonucleotide therapeutics, which have a basic skeleton of nucleic acids such as DNA and RNA, are highly specific because they act by binding to specific base sequences on genes, and are expected to be applied to the treatment of cancers and hereditary diseases that have been difficult to treat until now. Although there are various types of oligonucleotide therapeutics, oligonucleotide therapeutics composed of siRNA (small interfering RNA), which is a double-stranded RNA consisting of about twenty-odd base pairs, have been actively developed in recent years.

The double-stranded structure of nucleic acids is stabilized in a liquid phase by hydrogen bonds formed between base pairs and stacking interactions due to the overlapping of bases, but it is difficult to maintain this double-stranded structure in a gas phase. On the other hand, in mass spectrometry using Electrospray Ionization (ESI) or Matrix-Assisted Laser Desorption/Ionization (MALDI), sample molecules are ionized in a gas phase. Furthermore, in mass spectrometry using MALDI, the double strand is prone to separation due to the influence of the matrix and solvent used for preparing the analytical sample, the crystallization process, and other factors. For this reason, it has been difficult to detect double-stranded oligonucleotides such as siRNA by these mass spectrometry methods.

In response to such problems, Non-Patent Literature 1 describes that siRNA can be detected by subjecting an analytical sample prepared using a neutral matrix, instead of an acidic matrix that tends to act on hydrogen bonds, to MALDI mass spectrometry. Specifically, an analytical sample is prepared using 6-aza-2-thiothymine (ATT) as a matrix, ammonium citrate as a matrix additive, and water as a solvent, and the siRNA is analyzed by MALDI-TOFMS, which combines a MALDI ion source and a Time-of-Flight Mass Spectrometer (TOFMS).

### CITATION LIST

### PATENT LITERATURE

NON-PATENT LITERATURE 1 : Ute Bahr, et al., "Detection and Relative Quantification of siRNA Double Strands by MALDI Mass Spectrometry", Analytical Chemistry, 2008, Vol. 80, No. 16, pp. 6280-6285

### SUMMARY OF INVENTION

### TECHNICAL PROBLEM

In recent years, the use of MALDI-ITMS, which combines a MALDI ion source and an Ion Trap Mass Spectrometer (ITMS), has been attempted for the analysis of single-stranded oligonucleotides. However, it was not possible to analyze double-stranded oligonucleotides with MALDI-ITMS even under conditions that could analyze single-stranded oligonucleotides.

An object of the present invention is to provide a novel method that enables the analysis of double-stranded oligonucleotides using a MALDI ion trap mass spectrometer.

### SOLUTION TO PROBLEM

A method for analyzing a double-stranded oligonucleotide according to the present invention, which has been made to solve the above problem, is a method for analyzing a double-stranded oligonucleotide included in a sample by using an ion trap mass spectrometer equipped with an ion source based on matrix-assisted laser desorption/ionization, an ion trap unit for trapping and ejecting ions generated in the ion source, and a detection unit for detecting the ions ejected from the ion trap unit, the method comprising: introducing the sample into the ion source and irradiating the sample with a laser, trapping the ions generated in the ion source in the ion trap unit, and then ejecting and detecting the ions trapped in the ion trap unit, wherein a delay time, which is a time from irradiating the sample with the laser in the ion source until applying a trapping voltage for trapping ions in the ion trap unit, is set to be longer than a value preset for the ion trap mass spectrometer as a delay time for trapping ions having a mass-to-charge ratio comparable to a mass-to-charge ratio corresponding to a molecular weight of the double-stranded oligonucleotide.

### ADVANTAGEOUS EFFECTS OF INVENTION

According to the present invention, it is possible to analyze a double-stranded oligonucleotide using a MALDI ion trap mass spectrometer.

### BRIEF DESCRIPTION OF DRAWINGS

[FIG. 1] FIG. 1 is a schematic configuration diagram showing an example of a mass spectrometer (MALDI-ITMS) used in a method for analyzing a double-stranded oligonucleotide according to an embodiment of the present invention.
[FIG. 2] FIG. 2 shows mass spectra of vutrisiran under various setting conditions in Example 1 (Experimental Example 1-1).
[FIG. 3] FIG. 3 shows mass spectra of vutrisiran under other various setting conditions in Example 1 (Experimental Example 1-1).
[FIG. 4] FIG. 4 shows a mass spectrum of vutrisiran in Example 1 (Experimental Example 1-2).
[FIG. 5] FIG. 5 shows a mass spectrum of vutrisiran in Example 1 (Experimental Example 1-3).
[FIG. 6] FIG. 6 shows a mass spectrum of vutrisiran in Example 2 (Experimental Example 2-1).
[FIG. 7] FIG. 7 shows a mass spectrum of vutrisiran in Example 2 (Experimental Example 2-2).
[FIG. 8] FIG. 8 shows a mass spectrum of vutrisiran in Example 2 (Experimental Example 2-3).
[FIG. 9] FIG. 9 shows mass spectra of patisiran under various setting conditions in Example 3.
[FIG. 10] FIG. 10 shows mass spectra of patisiran under other various setting conditions in Example 3.
[FIG. 11] FIG. 11 shows mass spectra of patisiran under still other various setting conditions in Example 3.

### DESCRIPTION OF EMBODIMENTS

Hereinafter, an embodiment of a method for analyzing a double-stranded oligonucleotide according to the present invention will be described.

### <Mass Spectrometer>

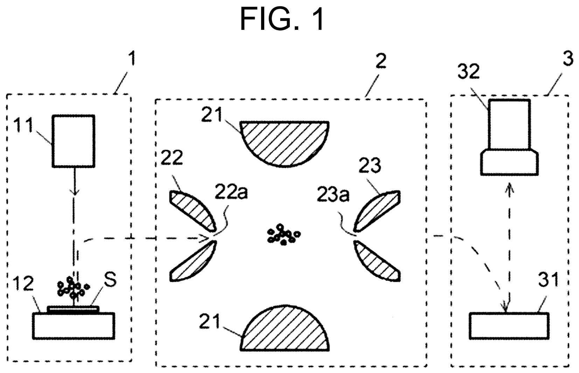

FIG. 1 shows a schematic configuration diagram of a mass spectrometer used in the present embodiment. This mass spectrometer is a MALDI-ITMS that combines a MALDI ion source and an ion trap mass spectrometer, and includes an ion source 1 that ionizes a sample containing an analyte, an ion trap 2 that temporarily traps ions of a predetermined mass-to-charge ratio (m/z) among the ions generated in the ion source 1 by the action of a high-frequency electric field and separates the trapped ions according to their mass-to-charge ratio, and a detection unit 3 that detects the separated ions.

### (Ion Source)

The ion source 1 is an ion source using the MALDI method, and includes a laser irradiation unit 11 that irradiates a sample with a laser beam, and a sample stage 12 on which a sample plate S carrying the sample is placed.

### (Ion Trap)

The ion trap 2 is a quadrupole ion trap including an annular ring electrode 21 and a pair of end cap electrodes 22 and 23 arranged opposite to each other with the ring electrode 21 interposed therebetween. An ion entrance hole 22a is formed in the entrance-side end cap electrode 22, and an ion exit hole 23a is formed in the exit-side end cap electrode 23.

A predetermined high-frequency voltage is applied to each of the ring electrode 21 and the end cap electrodes 22 and 23 of the ion trap 2. A high-frequency electric field is formed in the ion trap 2 to which the high-frequency voltage is applied, and thereby, ions can be trapped in the internal space surrounded by the ring electrode 21 and the end cap electrodes 22 and 23, or ions can be ejected from the internal space through the ion exit hole 23a. The ITMS in the present embodiment is a mass spectrometer that utilizes the mass separation function of the ion trap 2 itself to eject ions trapped in the ion trap 2 in ascending order of mass-to-charge ratio, and detects the ions with a detector 3 arranged outside the ion trap 2. Alternatively, the ITMS may be a mass spectrometer in which ions ejected all at once from the ion trap 2 are separated according to mass-to-charge ratio by a mass separator arranged outside the ion trap 2, such as a time-of-flight mass separator, and detected by a detector 3 arranged outside the ion trap 2.

The high-frequency voltage applied to the ring electrode 21 and the end cap electrodes 22 and 23 may be a sine wave voltage or a square wave voltage. An ion trap mass spectrometer that uses an electric field formed by a square wave voltage is called a Digital Ion Trap Mass Spectrometer (DITMS).

When the high-frequency voltage is a sine wave voltage, it is difficult to change the frequency because a resonator must be used in the voltage generator that generates the sine wave voltage. Therefore, the mass-to-charge ratio range of ions trapped in the ion trap 2 is controlled by changing the amplitude of the voltage.

On the other hand, when the high-frequency voltage is a square wave voltage, a square wave voltage can be generated by rapidly switching between two different voltages without using a resonator in the voltage generator. Therefore, in a DITMS, the mass-to-charge ratio range of ions trapped in the ion trap 2 can be controlled by changing the frequency while keeping the amplitude of the voltage constant. Specifically, a low mass cut-off (LMCO) value on the low mass-to-charge ratio side is set by adjusting the frequency of the high-frequency voltage, and the higher the frequency, the smaller the LMCO is set. Since there is an upper limit to the amount of ions that can be trapped in the ion trap 2, the mass-to-charge ratio range of ions trapped in the ion trap 2 (that is, the mass-to-charge ratio range measured by the DITMS) is set to the low mass-to-charge ratio side when the frequency is increased and the LMCO is set small, and is set to the high mass-to-charge ratio side when the frequency is decreased and the LMCO is set large.

### (Detector)

The detector 3 includes a conversion dynode 31 that converts ions into electrons, and a secondary electron multiplier 32 that multiplies the electrons arriving from the conversion dynode 31. A high voltage of the opposite polarity to the ions to be detected is applied to the conversion dynode 31, that is, a negative voltage for positive ions and a positive voltage for negative ions. The secondary electron multiplier 32 has multi-stage or continuous dynodes, and by applying a voltage to the secondary electron multiplier 32, secondary electrons emitted when electrons arriving from the conversion dynode 31 are incident are repeatedly multiplied. Thereafter, the increased secondary electrons are converted into a current signal and detected. For the voltages applied to the conversion dynode 31 and the secondary electron multiplier 32, device-specific specified values are preset according to the mass-to-charge ratio range of the ions trapped in the ion trap 2.

As a result of diligent studies, the inventors of the present application have found that in MALDI-ITMS, by appropriately setting the time from irradiating the sample with a laser beam in the ion source 1 to applying a high-frequency voltage to the ring electrode 21 of the ion trap 2 (hereinafter referred to as delay time), it is possible to detect a double-stranded oligonucleotide by mass spectrometry. Specifically, the delay time is set to a time longer than a value preset for the MALDI-ITMS as a delay time for trapping ions having a mass-to-charge ratio comparable to the mass-to-charge ratio corresponding to the molecular weight of the double-stranded oligonucleotide, which is the substance to be analyzed.

Generally, it is known that when a substance such as a peptide or a protein is analyzed by MALDI mass spectrometry, molecular-weight-related ions such as [M+H]⁺ or [M-H]⁻(M is a molecule, H is a hydrogen atom) are mainly detected. For example, when it is desired to detect [M+H]⁺ or [M-H]⁻ of a substance having a molecular weight of about 15,000, the delay time is usually set to about 25 µs. However, a double-stranded oligonucleotide having a similar molecular weight could not be detected with such a delay time. On the other hand, when the delay time was set to 80 µs, which is longer than the above value, it was confirmed that a peak derived from the double-stranded oligonucleotide was detected. Generally, as the delay time becomes longer, ions on the higher mass-to-charge ratio side are preferentially trapped in the ion trap 2, but the above-mentioned delay time value (80 µs) is a value for preferentially trapping ions with a much larger mass-to-charge ratio than [M+H]⁺ or [M-H]⁻ of a substance with a molecular weight of about 15,000. In other words, a delay time of 80 µs was a value exceeding the range assumed from the conventionally known relationship between the mass-to-charge ratio of the detected ions and the delay time.

The delay time can be set to an appropriate value according to the molecular weight of the double-stranded oligonucleotide. For example, by setting the delay time to a value in the range of 40 ~ 80 µs, a double-stranded oligonucleotide with a molecular weight of 10,000 or more can be detected with high sensitivity. From the viewpoint of improving sensitivity, it is more preferable to set it in the range of 60 ~ 80 µs. Considering that a typical delay time for preferentially trapping ions with a mass-to-charge ratio of about 20,000 to 70,000 in the ion trap 2 is about 50 µs, it is understood that the above-mentioned range of delay times is unexpected. In this specification, a numerical range from a lower limit value to an upper limit value is indicated using the symbol "~" as in "(lower limit value) ~ (upper limit value)", but this numerical range includes the lower limit value itself and the upper limit value itself.

In addition to setting the delay time, at least one of the voltage applied to the conversion dynode 31 of the detector 3 and the voltage applied to the secondary electron multiplier 32 may be made larger than the device-specific specified value so that the signal intensity of the ions detected by the detector 3 increases. This allows for the detection of double-stranded oligonucleotides with higher sensitivity. Hereinafter, the voltage applied to the conversion dynode 31 is referred to as the dynode voltage, and the voltage applied to the secondary electron multiplier 32 is referred to as the detector voltage. The dynode voltage is preferably set to be about 5 to 30% higher than the device-specific specified value. Also, the detector voltage is preferably set to be about 5 to 50% higher than the device-specific specified value. For example, the dynode voltage is preferably set to about 8000 V when the specified value is 7000 V, and the detector voltage is preferably set to about 1600 to 2000 V when the specified value is 1300 V.

### <Analytical Sample>

The analytical sample is prepared by mixing a sample solution containing a double-stranded oligonucleotide and a matrix solution containing a matrix, and drying the mixed solution on the sample plate S of the mass spectrometer. At this time, a mixed solution in which the sample solution and the matrix solution are mixed in advance may be prepared, and the mixed solution may be dropped onto the sample plate S and dried, or the sample solution and the matrix solution may be dropped separately onto the sample plate S, mixed on the sample plate S, and dried.

### (Double-stranded Oligonucleotide)

The double-stranded oligonucleotide, which is the analyte in this embodiment, may be either DNA or RNA. The length of the double-stranded oligonucleotide is not particularly limited, and is, for example, about several tens of base pairs. The double-stranded oligonucleotide may be a chemically synthesized artificial nucleic acid such as Oligonucleotide therapeutics, or a natural product obtained from an organism or a processed product thereof. The analyte, the double-stranded oligonucleotide, may have an unknown or known molecular weight. If the structure of the double-stranded oligonucleotide is known, the molecular weight can be calculated from its chemical formula. Alternatively, the molecular weight may have been measured in advance by other methods other than mass spectrometry, such as electrophoresis. For example, when the double-stranded oligonucleotide is siRNA, its molecular weight is about 1,000 to 30,000.

### (Matrix)

As the matrix, an appropriate substance can be selected according to the type of double-stranded oligonucleotide. Examples include 3-hydroxypicolinic acid (3-HPA), 2,4-dihydroxyacetophenone (2,4-DHAP), 2,5-dihydroxybenzoic acid (DHB), 2',4',6'-trihydroxyacetophenone monohydrate (THAP), 6-aza-2-thiothymine (ATT), 3-aminopyrazine-2-carboxylic acid (APCA), anthranilic acid (AA), and nicotinic acid (NA). From the viewpoint of being able to detect double-stranded nucleotides with high sensitivity, 3-HPA, DHAP, and THAP are preferable as the matrix, and 3-HPA is particularly preferable.

The matrix solution may be a mixed matrix solution containing two or more types of matrices. The mixed matrix solution preferably contains at least 3-HPA, and more preferably contains 3-HPA and THAP or 3-HPA and 2,4-DHAP.

The concentration of the matrix in the matrix solution is not particularly limited, but is preferably 30 to 50 mg/mL from the viewpoint of being able to detect [M+H]⁺ or [M-H]⁻of the double-stranded oligonucleotide with high sensitivity.

### (Matrix Additive)

The matrix solution may further contain a matrix additive. As the matrix additive, ammonium citrate dibasic (ACD) can be used. There are several types of ammonium salts of citric acid depending on the number of ammonium ions bonded to one citrate ion, but the one preferably used in this embodiment is a salt in which two ammonium ions are bonded to one citrate ion.

The concentration of the matrix additive in the matrix solution is not particularly limited, but is preferably 70 to 200 mM, and more preferably 100 to 200 mM, from the viewpoint of being able to detect [M+H]⁺ or [M-H]⁻ of the double-stranded oligonucleotide with high sensitivity.

### (Solvent)

Since the double-stranded oligonucleotide is water-soluble and its double-stranded structure is stabilized by hydrogen bonds in the liquid phase, it is preferable to use water as the solvent for the sample solution. The solvent for the matrix solution preferably contains water, and may also contain a polar solvent such as acetonitrile.

Hereinafter, the method for analyzing a double-stranded oligonucleotide according to the present invention will be described by way of examples, but these are merely illustrative and the present invention is not limited thereto.

### EXAMPLE 1

### (Experimental Example 1-1)

### <1. Preparation of Sample Solution>

As a sample solution, a 20 pmol/µL aqueous solution of vutrisiran (siRNA (double-stranded RNA consisting of a sense strand and an antisense strand, MW 16344.55 Da, synthetic nucleic acid for research manufactured by MedchemExpress)) was prepared. (Sense strand: 5'-Ums-Gms-Gm-Gm-Am-Um-Uf-Um-Cf-Af-Uf-Gm-Um-Am-Am-Cm-Cm-Am-Am-Gm-Am-R1-3' (Af=2'-fluoro adenosine, Am=2'-O-methyladenosine, Cf=2'-fluorocytidine, Cm=2'-O-methylcytidine, Gm=2'-O-methylguanosine, Uf=2'-fluorouridine, Um=2'-O-methyluridine, R1=3-branched N-acetylgalactosamine (GalNAc), s-(hyphen)=phosphorothioate linkage), SEQ ID NO: 1, MW 8788.55 Da) (Antisense strand: 3'-Cms-Ums-Am-Cm-Cm-Cm-Um-Af-Am-Af-Gm-Um-Am-Cm-Af-Um-Um-Gf-Gm-Um-Ums-Cfs-Um-5' (Af=2'-fluoro adenosine, Am=2'-O-methyladenosine, Cf=2'-fluorocytidine, Cm=2'-O-methylcytidine, Gf=2'-fluoroguanosine, Gm=2'-O-methylguanosine, Um=2'-O-methyluridine, s-(hyphen)=phosphorothioate linkage), SEQ ID NO: 2, MW 7556.00 Da).

### <2. Preparation of Matrix Solution>

Ammonium citrate dibasic (ACD) was used as a matrix additive and dissolved in a 50% aqueous acetonitrile solution to a concentration of 200 mM to prepare an ACD solution. To this ACD solution, 3-hydroxypicolinic acid (3-HPA) was dissolved as a matrix to a concentration of 40 mg/mL to prepare a matrix solution.

### <3. Preparation of Analytical Sample>

The sample solution prepared in 1. and the matrix solution prepared in 2. were mixed at a 1:1 (v/v) ratio, and 1 µL of the resulting mixed solution was dropped onto a sample plate (SUS plate) and dried.

### <4. Mass Spectrometry>

For mass spectrometry, a MALDI digital ion trap mass spectrometer (MALDI-DITMS, manufactured by Shimadzu Corporation, trade name: MALDImini-1) was used. The sample plate from 3. was inserted into the MALDI-DITMS, and measurement was performed in positive mode using the raster function. As the measurement conditions, measurement mode 3 (m/z 2000-18000), which is a mass-to-charge ratio range matching the molecular weight of the siRNA contained in the analytical sample, was used, and measurement was performed using the optimal laser power (threshold value) under the device-specific setting conditions preset according to the measurement mode (detector voltage (DV1): 1300 (V), dynode voltage (DV2): 7000 (V), delay time (RF delay: RF): 25 (µs)). In addition, measurements were performed by appropriately changing the detector voltage (DV1), dynode voltage (DV2), and delay time (RF).

### <Results>

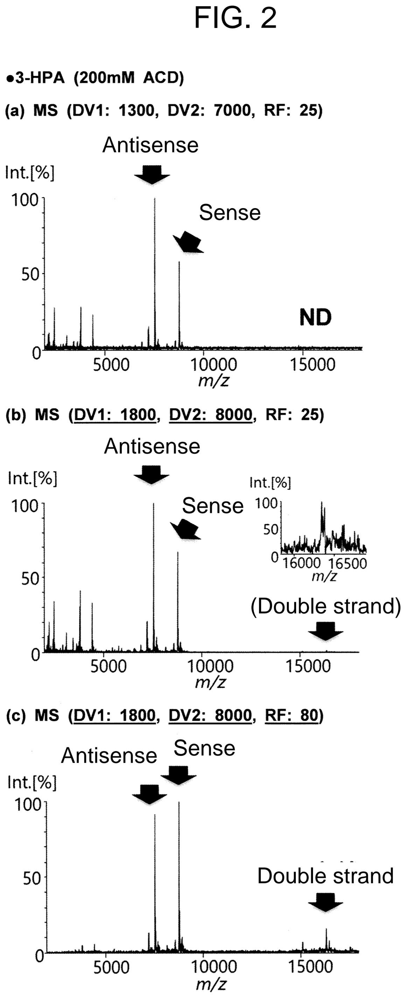

FIG. 2 shows the mass spectrum of vutrisiran when 3-HPA was used as the matrix.

When measured under the device-specific setting conditions (DV1: 1300, DV2: 7000, RF: 25), mainly two peaks derived from the sense strand and the antisense strand were both detected as [M+H]⁺, but no peak derived from the double strand was detected (FIG. 2(a)). On the other hand, when measured under conditions that amplified the signal intensity by increasing the values of the detector voltage (DV1) and the dynode voltage (DV2) (DV1: 1800, DV2: 8000, RF: 25), a peak derived from the double strand was detected as [M+H]⁺, but its sensitivity was low (FIG. 2(b)). Furthermore, when measured under a condition with an increased delay time (RF) value (DV1: 1800, DV2: 8000, RF: 80), the peak derived from the double strand was detected with high sensitivity (FIG. 2(c)).

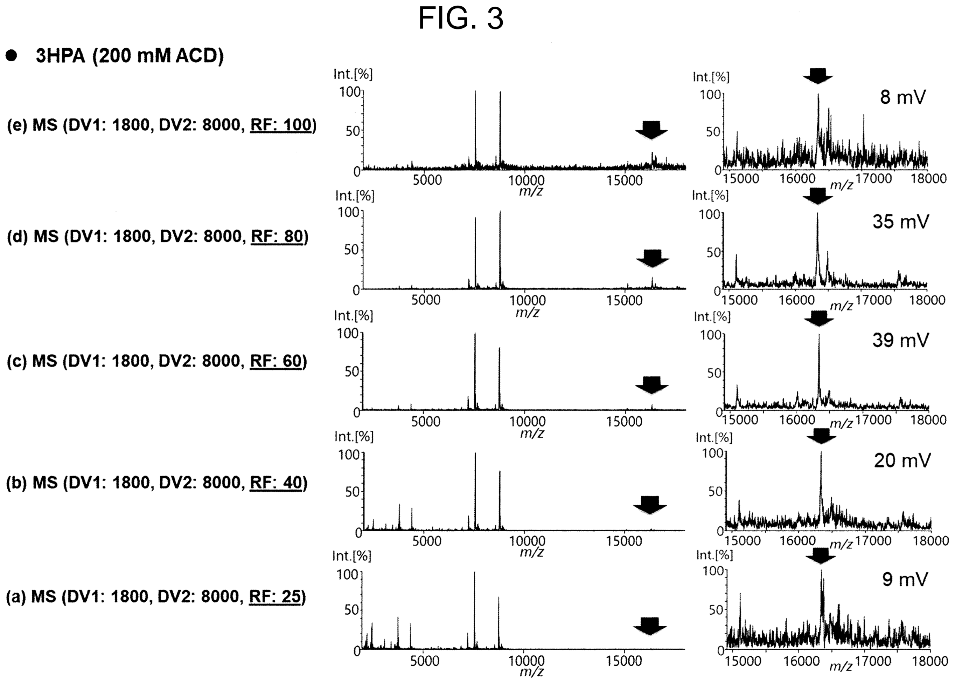

FIG. 3 shows mass spectra of vutrisiran when 3-HPA was used as the matrix, the detector voltage was set to DV1: 1800, the dynode voltage was set to DV2: 8000, and the value of the delay time (RF) was varied. From the bottom, the mass spectra are for RF: 25, 40, 60, 80, 100, respectively, and on the right side of each stage, an enlarged view of the vicinity of the m/z of the peak derived from the double strand is shown. The arrows in the figure indicate the detection status of the peak derived from the double strand, and the numerical value in the enlarged view indicates the peak intensity (mV) of the peak.

As shown in FIG. 3, the peak derived from the double strand was detected at all delay time (RF) values, but the noise was large and the peak intensity was low at RF: 25 and 100 (FIG. 3(a), (e)). On the other hand, the noise was low at RF: 40-80 (FIG. 3(b)-(d)), and the peak derived from the double strand was detected with high sensitivity, especially at RF: 60 and 80 (FIG. 3(c), (d)).

### (Experimental Example 1-2)

Mass spectrometry was performed in the same manner as in Experimental Example 1-1 of Example 1, except for the method of preparing the matrix solution.

### <2. Preparation of Matrix Solution>

ACD as a matrix additive was dissolved in a 50% aqueous acetonitrile solution to a concentration of 200 mM to prepare an ACD solution. To this ACD solution, 3-HPA, 2,4-dihydroxyacetophenone (DHAP), or 2',4',6'-trihydroxyacetophenone monohydrate (THAP) was dissolved as a matrix to a concentration of 40 mg/mL to prepare three types of matrix solutions (3-HPA solution, DHAP solution, THAP solution).

The 3-HPA solution and the THAP solution were mixed at 1:1 (v/v) and 1:3 (v/v) to prepare two types of mixed matrix solutions (3-HPA/THAP (1:1) solution, 3-HPA/THAP (1:3) solution).

### <Results>

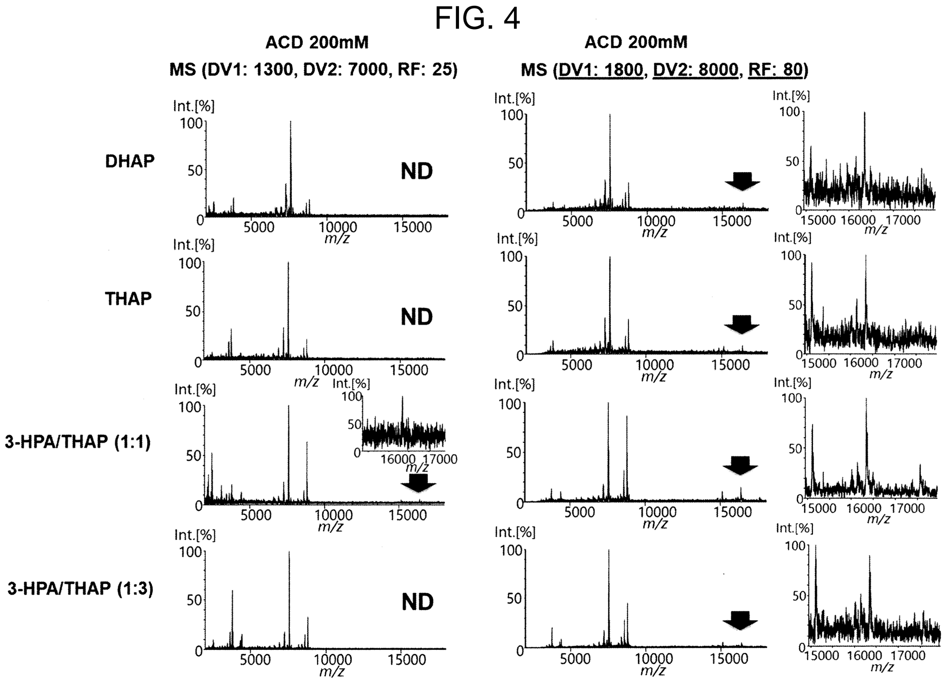

FIG. 4 shows mass spectra of vutrisiran using various matrices (DHAP, THAP, 3-HPA/THAP (1:1), 3-HPA/THAP (1:3)). The mass spectra on the left side were measured using the device-specific setting conditions (DV1: 1300, DV2: 7000, RF: 25), and the mass spectra on the right side were measured using the conditions under which the peak derived from the double strand was detected with high sensitivity in Experimental Example 1-1 (DV1: 1800, DV2: 8000, RF: 80) (hereinafter, this setting condition is referred to as the double-strand detection setting condition). The arrows in the figure indicate the detection status of the peak derived from the double strand, and ND indicates not detected. For those where the peak derived from the double strand was detected, an enlarged view of the vicinity of the m/z of the peak is also shown.

Similar to the case where 3-HPA was used as the matrix, under the conditions where the values of the detector voltage (DV1), the dynode voltage (DV2), and the delay time (RF) were made larger than the device-specific setting conditions, a peak derived from the double strand was detected regardless of which matrix was used. From FIGS. 2-4, it was confirmed that siRNA was detected with the highest sensitivity when 3-HPA was used as the matrix.

### (Experimental Example 1-3)

Mass spectrometry was performed in the same manner as in Experimental Example 1-1 of Example 1, except for the method of preparing the matrix solution.

### <2. Preparation of Matrix Solution>

ACD as a matrix additive was dissolved in a 50% aqueous acetonitrile solution to each concentration (70, 100, 200, 300, 400 mM) to prepare five types of ACD solutions. To each of these five ACD solutions, 3-HPA was dissolved as a matrix to a concentration of 40 mg/mL to prepare five types of matrix solutions.

### <Results>

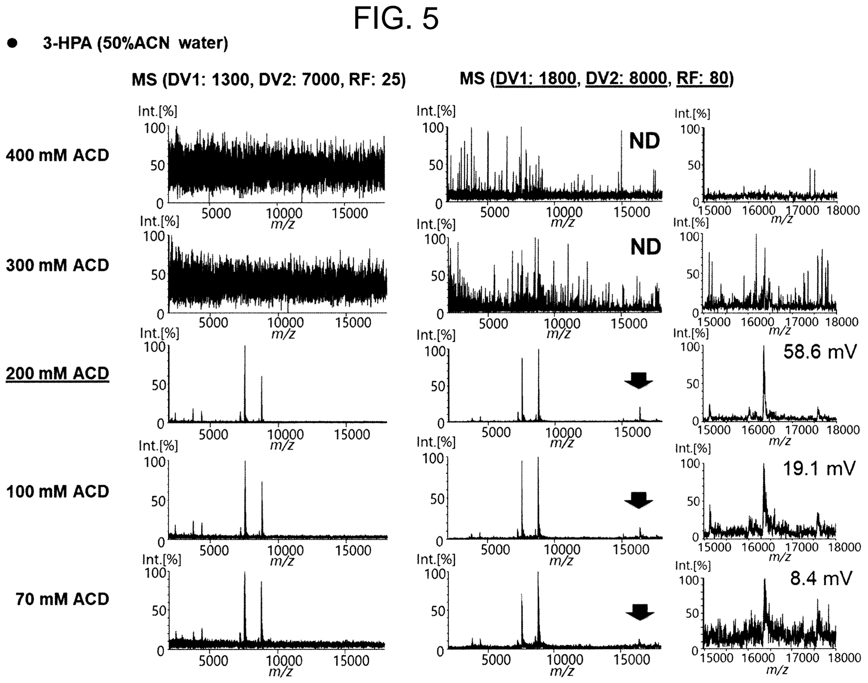

FIG. 5 shows mass spectra of vutrisiran when 3-HPA was used as the matrix and the ACD concentration in the matrix solution was varied. From the bottom, the mass spectra are at ACD concentrations of 70, 100, 200, 300, and 400 mM, and for each, the left side was measured using the device-specific setting conditions, and the right side was measured using the double-strand detection setting conditions. The mass spectra on the right side further show an enlarged view of the vicinity of the m/z of the peak derived from the double strand, and for those where the peak derived from the double strand was detected, its peak intensity (mV) is shown.

As shown in FIG. 5, when the ACD concentration was 70 to 200 mM, a peak derived from the double strand was detected under the double-strand detection setting conditions. In addition, the peak intensity of the peak derived from the double strand was higher when using the matrix solution with an ACD of 200 mM than when using the matrix solution with an ACD of 70 mM.

Although detailed data are not shown here, an ACD concentration of 70 mM was the optimal concentration condition for the analysis of single-stranded oligonucleotides. It has been reported that double-stranded oligonucleotides contain more sodium ions (Na⁺) in the sample than single-stranded oligonucleotides, and that a large amount of this Na⁺ inhibits the ionization of the sample. In this experimental example, the reason why the peak intensity of the peak derived from the double strand was higher when the ACD concentration was 200 mM than when it was 70 mM is considered to be, as one reason, that the higher the concentration of ACD, which is thought to have the role of capturing Na⁺, the less likely ionization is inhibited.

From the above, it was confirmed that to detect double-stranded oligonucleotides, it is effective to set the detector voltage (DV1), dynode voltage (DV2), and delay time (RF) values higher than the device-specific setting conditions, and under such double-strand detection setting conditions, a peak derived from the double strand can be detected with multiple matrices. 3-HPA was most preferable as the matrix. In addition, it was confirmed that as for the concentration of the matrix additive, ammonium citrate dibasic, a peak derived from the double strand is detected with higher sensitivity at a concentration higher than the optimal concentration for the analysis of single-stranded oligonucleotides.

### EXAMPLE 2

### (Experimental Example 2-1)

Mass spectrometry was performed in the same manner as in Experimental Example 1-1 of Example 1, except for the method of preparing the matrix solution.

### <2. Preparation of Matrix Solution>

ACD as a matrix additive was dissolved in a 50% aqueous acetonitrile solution to a concentration of 200 mM to prepare an ACD solution. To this ACD solution, 3-HPA or 6-aza-2-thiothymine (ATT) was dissolved as a matrix to a concentration of 40 mg/mL to prepare two types of matrix solutions (3-HPA solution, ATT solution).

### <Results>

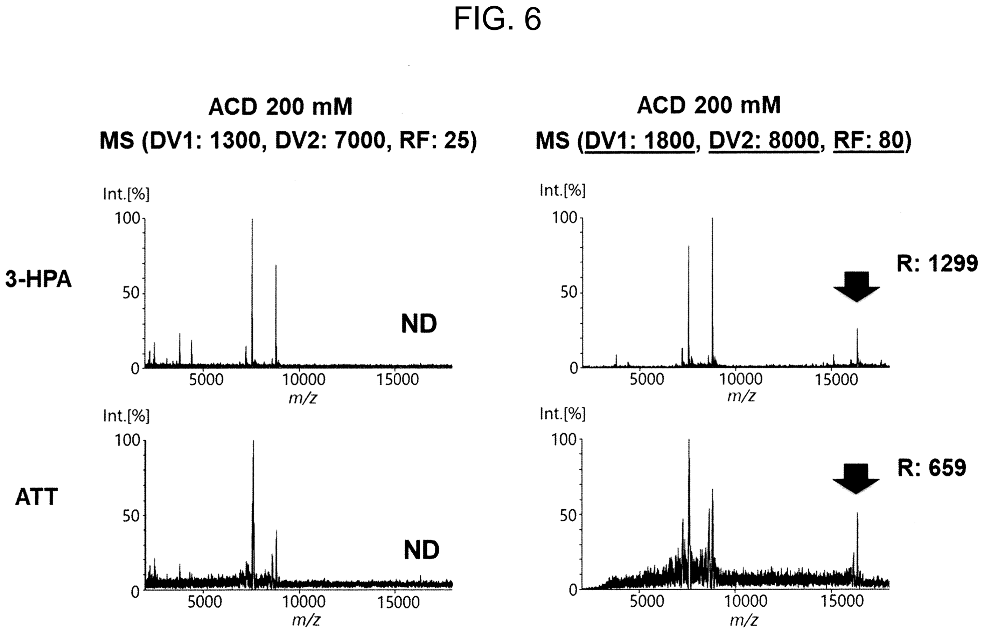

FIG. 6 shows mass spectra of vutrisiran when 3-HPA or ATT was used as the matrix. The upper panel shows the mass spectrum when 3-HPA was used, and the lower panel shows the mass spectrum when ATT was used, and for each, the left side was measured using the device-specific setting conditions, and the right side was measured using the double-strand detection setting conditions. The numerical value indicated by R in the figure shows the resolution of the peak derived from the double strand.

Non-Patent Literature 1 describes that the double-strand peak is more easily detected when using the neutral matrix ATT compared to the acidic matrix 3-HPA. However, in this experimental example, although a peak derived from the double strand was detected under the double-strand detection setting conditions for both matrices, the mass spectrum when ATT was used had more noise and lower peak resolution compared to the mass spectrum when 3-HPA was used, and the quality of the mass spectrum as a whole was low.

### (Experimental Example 2-2)

Mass spectrometry was performed in the same manner as in Experimental Example 1-1 of Example 1, except for the method of preparing the matrix solution.

### <2. Preparation of Matrix Solution>

ACD as a matrix additive was dissolved in a 50% aqueous acetonitrile solution or water to a concentration of 200 mM to prepare two types of ACD solutions. To each of these two ACD solutions, 3-HPA was dissolved as a matrix to a concentration of 40 mg/mL to prepare two types of matrix solutions (3-HPA solution, 3-HPA aqueous solution).

### <Results>

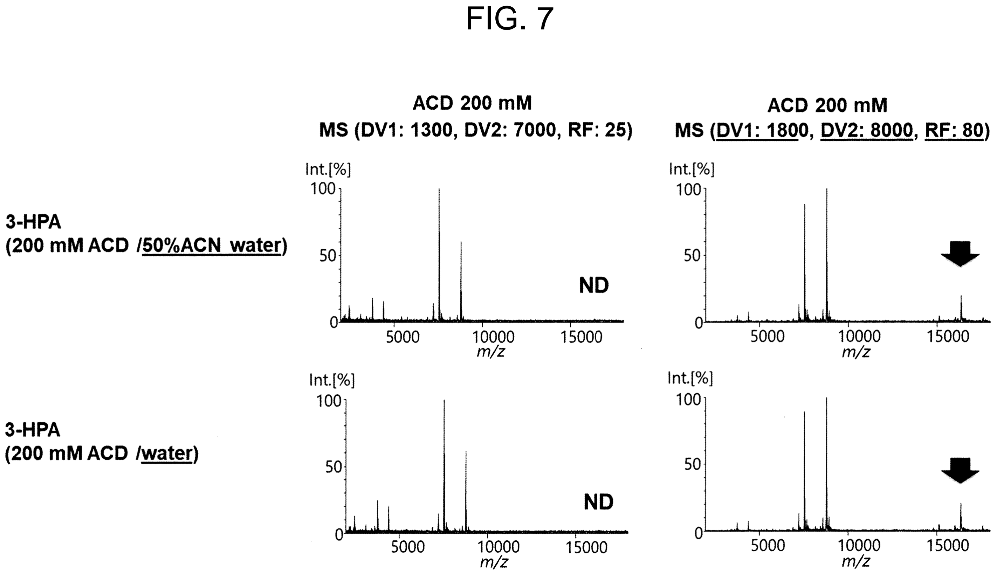

FIG. 7 shows mass spectra of vutrisiran when 3-HPA was used as the matrix and a 50% aqueous acetonitrile solution or water was used as the solvent for the matrix solution. The upper panel shows the mass spectrum when a 50% aqueous acetonitrile solution was used, and the lower panel shows the mass spectrum when water was used, and for each, the left side was measured using the device-specific setting conditions, and the right side was measured using the double-strand detection setting conditions.

According to Non-Patent Literature 1, acetonitrile enhances the solubility of the matrix but suppresses the detection of double-stranded oligonucleotides. However, in this experimental example, it was confirmed that a peak derived from the double strand was detected with high sensitivity under the double-strand detection setting conditions, regardless of whether a 50% aqueous acetonitrile solution or water was used as the solvent.

### (Experimental Example 2-3)

Mass spectrometry was performed in the same manner as in Experimental Example 1-1 of Example 1, except for the method of preparing the matrix solution.

### <2. Preparation of Matrix Solution>

ACD as a matrix additive was dissolved in a 50% aqueous acetonitrile solution to a concentration of 200 mM to prepare an ACD solution. To this ACD solution, 3-HPA was dissolved as a matrix to a concentration of 40 mg/mL to prepare a matrix solution (3-HPA solution).

Also, ACD was dissolved in water to a concentration of 100 mM or 200 mM to prepare two types of aqueous ACD solutions, and to each of these two aqueous ACD solutions, 3-HPA was dissolved to a concentration of 40 mg/mL to prepare two types of matrix solutions (3-HPA aqueous solution).

ACD as a matrix additive was dissolved in a 50% aqueous acetonitrile solution to a concentration of 200 mM to prepare an ACD solution. To this ACD solution, ATT was dissolved as a matrix to a concentration of 40 mg/mL to prepare a matrix solution (ATT solution).

Also, ACD was dissolved in water to a concentration of 100 mM to prepare an aqueous ACD solution, and to this aqueous ACD solution, ATT was dissolved to a concentration of 6 mg/mL to prepare a matrix solution (aqueous ATT solution).

### <Results>

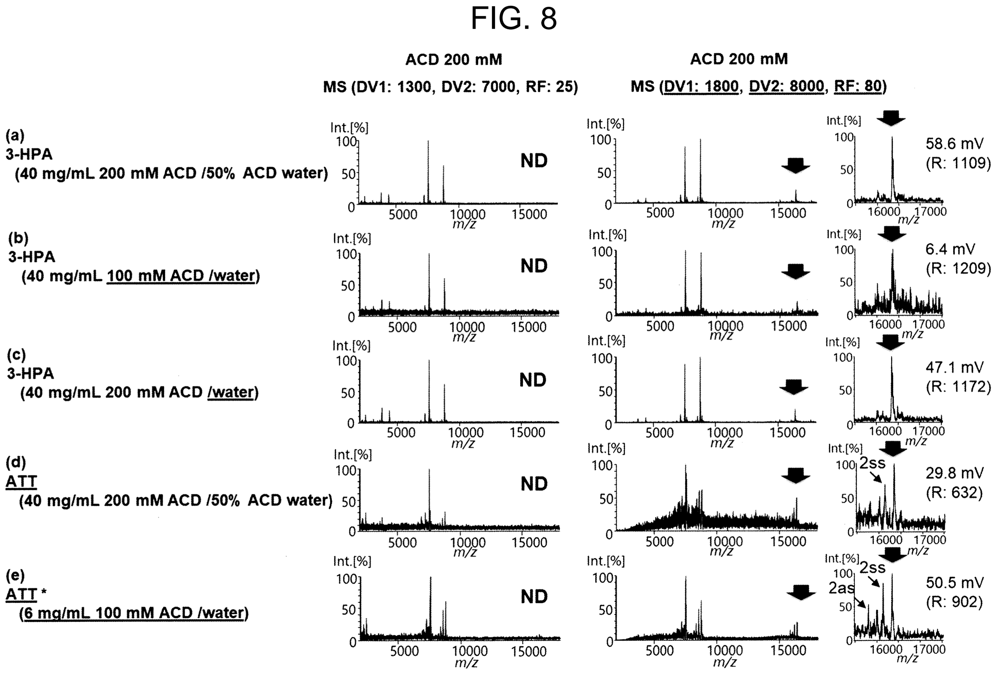

FIG. 8 shows mass spectra of vutrisiran using various matrix solutions containing 3-HPA or ATT as a matrix. The left side was measured using the device-specific setting conditions, and the right side was measured using the double-strand detection setting conditions. The mass spectra on the right side further show an enlarged view of the vicinity of the m/z of the peak derived from the double strand, and the numerical values in the enlarged view indicate the peak intensity (mV) and resolution (R) of the peak.

When ATT was used as the matrix (FIG. 8(d), (e)), a peak derived from the double strand was detected, but the noise was high and the resolution was low. In addition, in FIGS. 8(d) and (e), peaks derived from dimers of the sense strand and the antisense strand (in the enlarged view, the peak derived from the dimer of the sense strand is labeled 2ss, and the peak derived from the dimer of the antisense strand is labeled 2as) and multiple peaks derived from base-loss ions were detected. Note that the condition of the matrix solution in FIG. 8(e) is the same as the condition under which siRNA was detected by MALDI-TOFMS in Non-Patent Literature 1. In contrast, when 3-HPA was used (FIG. 8(a)-(c), especially FIG. 8(a), (c)), the double-strand peak was detected with high sensitivity and high resolution. Also, in FIGS. 8(a)-(c), no peaks derived from dimers were detected, and there were few peaks derived from base-loss ions, resulting in a relatively simple mass spectrum.

From the above, it was confirmed that when measurement was performed by setting the MALDI-DITMS to the double-strand detection setting conditions, a better mass spectrum was obtained using the acidic matrix 3-HPA than using the neutral matrix ATT, which is recommended in Non-Patent Literature 1.

### EXAMPLE 3

### <1. Preparation of Sample Solution>

As a sample solution, a 20 pmol/µL aqueous solution of patisiran (siRNA (double-stranded RNA consisting of a sense strand and an antisense strand), MW 13424 Da, synthetic nucleic acid for research manufactured by GeneDesign, Inc.) was prepared. (Sense strand: 5'-G-Um-A-A-Cm-Cm-A-A-G-A-G-Um-A-Um-Um-Cm-Cm-A-Um-dT-dT-3' (dT=thymidine deoxyribonucleotide, Cm=2'-O-methylcytidine, Um=2'-O-methyluridine), SEQ ID NO: 3, MW 6764 Da) (Antisense strand: 3'-dT-dT-C-A-Um-U-G-G-U-U-C-U-C-A-Um-A-A-G-G-U-A-5' (dT=thymidine deoxyribonucleotide, Cm=2'-O-methylcytidine, Um=2'-O-methyluridine), SEQ ID NO: 4, MW 6660 Da).

### <2. Preparation of Matrix Solution>

ACD as a matrix additive was dissolved in a 50% aqueous acetonitrile solution to a concentration of 70 mM or 200 mM to prepare two types of ACD solutions. To each of these two ACD solutions, 3-HPA was dissolved as a matrix to a concentration of 40 mg/mL to prepare two types of matrix solutions.

### <3. Preparation of Analytical Sample>

The sample solution prepared in 1. and the matrix solution prepared in 2. were mixed at a 1:1 (v/v) ratio, and 1 µL of the resulting mixed solution was dropped onto a sample plate (SUS plate) and dried.

### <4. Mass Spectrometry>

For mass spectrometry, a MALDI digital ion trap mass spectrometer (MALDI-DITMS, manufactured by Shimadzu Corporation, trade name: MALDImini-1) was used. The sample plate from 3. was inserted into the MALDI-DITMS, and measurement was performed in positive mode using the raster function. As the measurement conditions, measurement mode 3 (m/z 2000-18000), which is a mass-to-charge ratio range matching the molecular weight of the siRNA contained in the analytical sample, was used, and measurement was performed using the optimal laser power (threshold value) with the device's specified setting conditions: detector voltage (DV1): 1300 (V), dynode voltage (DV2): 7000 (V), delay time (RF): 25 (µs). In addition, measurements were performed by appropriately changing the detector voltage (DV1), dynode voltage (DV2), and delay time (RF).

### <Results>

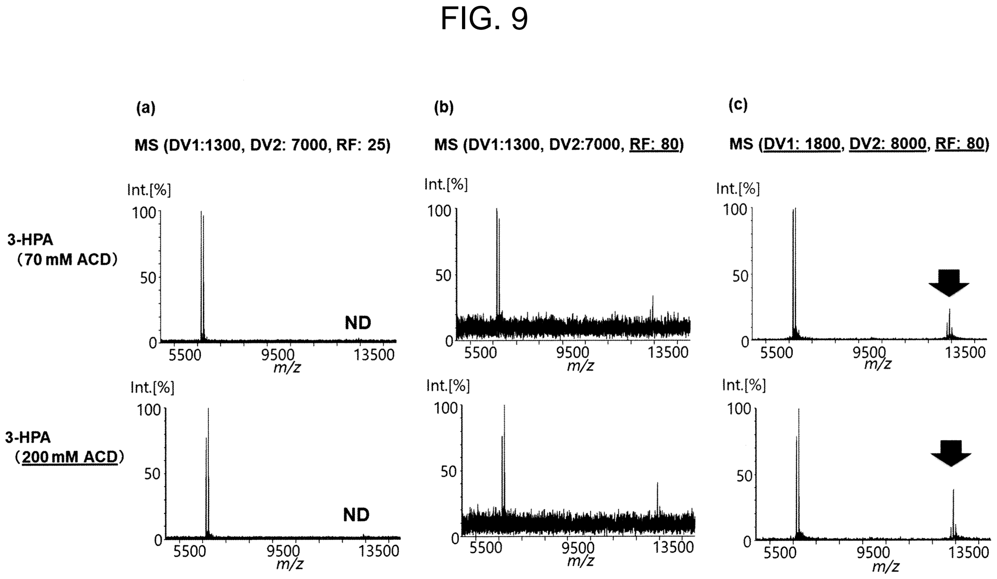

FIG. 9 shows mass spectra of patisiran under various setting conditions when 3-HPA was used as the matrix and the ACD concentration in the matrix solution was varied. The upper panel shows the mass spectrum at an ACD concentration of 70 mM, and the lower panel shows the mass spectrum at an ACD concentration of 200 mM.

In both cases of ACD 70 mM and 200 mM, when measured under the device-specific setting conditions (DV1: 1300, DV2: 7000, RF: 25), mainly two peaks derived from the sense strand and the antisense strand were both detected as [M+H]⁺, but no peak derived from the double strand was detected (FIG. 9(a)). On the other hand, when measured under a condition with an increased delay time (RF) value (DV1: 1300, DV2: 7000, RF: 80), a peak derived from the double strand was detected as [M+H]⁺ in both cases of ACD 70 mM and 200 mM. However, the signal-to-noise ratio (S/N) was low (FIG. 9(b)). Furthermore, when measured under a condition with increased detector voltage (DV1) and dynode voltage (DV2) values (DV1: 1800, DV2: 8000, RF: 80), the peak derived from the double strand was detected with high sensitivity in both cases of ACD 70 mM and 200 mM (FIG. 9(c)). In FIG. 9(c), the sensitivity was higher at 200 mM ACD than at 70 mM ACD. From the above, it was confirmed that by adjusting not only the delay time but also the detector voltage and the dynode voltage to be larger than the values of the device-specific setting conditions, the peak derived from the double strand can be detected with higher sensitivity.

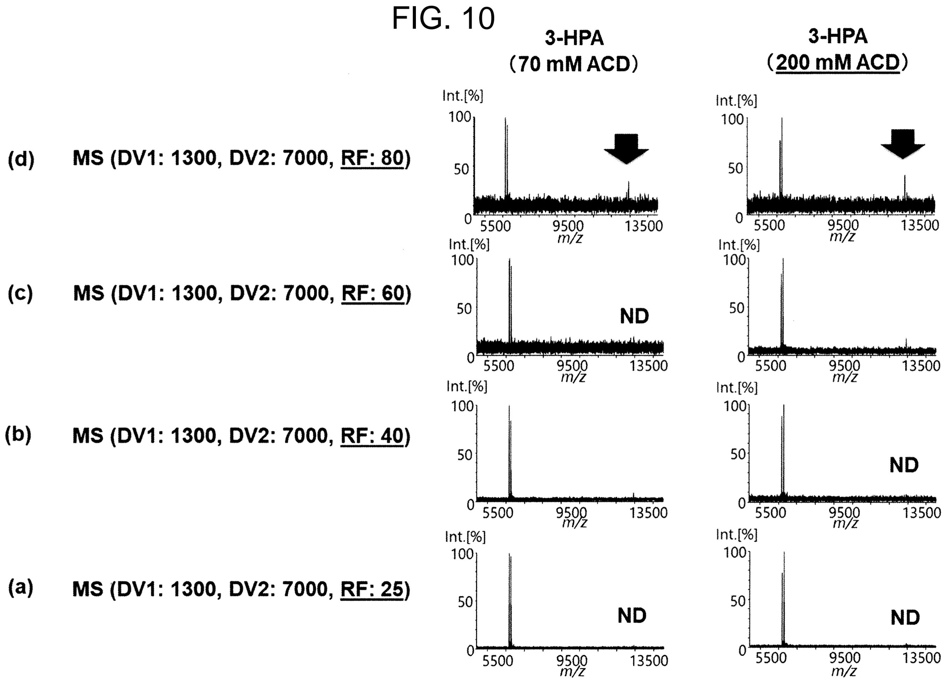

FIG. 10 shows mass spectra of patisiran when the detector voltage (DV1) and dynode voltage (DV2) were not changed from the device-specific setting conditions (DV1: 1300, DV2: 7000), and only the delay time (RF) was varied, using 3-HPA as the matrix. From the bottom, the RF values are: 25, 40, 60, 80, and for each, the left side is the mass spectrum under the condition of 70 mM ACD, and the right side is the mass spectrum under the condition of 200 mM ACD.

From FIG. 10, it was confirmed that when the delay time value was increased without changing the detector voltage and dynode voltage values, a peak derived from the double strand came to be detected, but the S/N decreased as the delay time value increased.

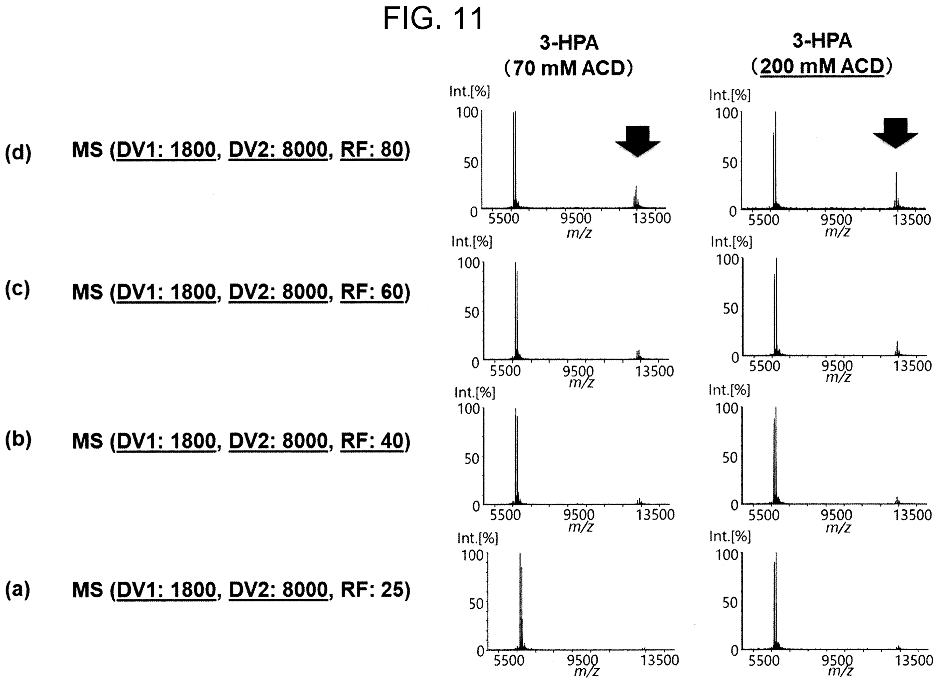

On the other hand, FIG. 11 shows mass spectra of patisiran when the detector voltage (DV1) and dynode voltage (DV2) values were changed to values larger than the device-specific setting conditions (DV1: 1800, DV2: 8000), and the delay time (RF) value was varied. From the bottom, the RF values are: 25, 40, 60, 80, and for each, the left side is the mass spectrum at 70 mM ACD, and the right side is the mass spectrum at 200 mM ACD.

From FIG. 11, when the detector voltage and dynode voltage values were increased, the S/N did not decrease significantly even when the delay time value was increased. As a result, the peak derived from the double strand was detected with the highest sensitivity at RF: 80, and at this RF value, the sensitivity was higher at 200 mM ACD than at 70 mM ACD. In addition, it was confirmed that by adjusting not only the delay time but also the detector voltage and dynode voltage values to be larger than the values of the device-specific setting conditions, the peak derived from the double strand is detected with higher sensitivity.

### [Aspects]

It will be apparent to those skilled in the art that the exemplary embodiments described above are specific examples of the following aspects.

(Item 1) A method for analyzing a double-stranded oligonucleotide according to one aspect of the present invention is a method for analyzing a double-stranded oligonucleotide included in a sample by using an ion trap mass spectrometer comprising an ion source based on matrix-assisted laser desorption/ionization, an ion trap unit for trapping and ejecting ions generated in the ion source, and a detection unit for detecting the ions ejected from the ion trap unit, the method comprising: introducing the sample into the ion source and irradiating the sample with a laser, trapping the ions generated in the ion source in the ion trap unit, and then ejecting and detecting the ions trapped in the ion trap unit, wherein a delay time, which is a time from irradiating the sample with the laser in the ion source until applying a trapping voltage for trapping ions in the ion trap unit, is set to be longer than a value preset for the ion trap mass spectrometer as a delay time for trapping ions having a mass-to-charge ratio comparable to a mass-to-charge ratio corresponding to a molecular weight of the double-stranded oligonucleotide.

Generally, in analysis using an ion trap mass spectrometer, the relationship between the mass-to-charge ratio of the ions to be detected and the delay time is known from experience. The "preset value" refers to a value that is preset for the mass spectrometer as a delay time for trapping ions having a mass-to-charge ratio comparable to the mass-to-charge ratio of [M+H]⁺ or [M-H]⁻ corresponding to the molecular weight of the double-stranded oligonucleotide, based on the empirically known relationship between the mass-to-charge ratio of the ions to be detected and the delay time.

The analyte, the double-stranded oligonucleotide, may have an unknown or known molecular weight. When the molecular weight of the double-stranded oligonucleotide as the analyte is known, the presence and molecular weight information of the double-stranded oligonucleotide can be confirmed by whether or not a peak providing the molecular weight information is detected, by performing mass spectrometry in a mass range that includes the mass-to-charge ratio corresponding to the molecular weight, under a condition where the delay time is set longer than a preset value. On the other hand, when the molecular weight of the double-stranded oligonucleotide as the analyte is unknown, the presence and molecular weight information of the double-stranded oligonucleotide can be confirmed by whether or not a peak providing the molecular weight information is detected, by performing mass spectrometry in each of several candidate mass ranges that include the mass-to-charge ratio corresponding to the assumed molecular weight of the double-stranded oligonucleotide, under a condition where the delay time is set longer than a preset value.

According to the method for analyzing a double-stranded oligonucleotide according to Item 1, a double-stranded oligonucleotide can be analyzed by a MALDI ion trap mass spectrometer.

(Item 2) In the method for analyzing a double-stranded oligonucleotide according to Item 1, the detection unit may comprise a conversion dynode and a secondary electron multiplier, and at least one of a voltage applied to the conversion dynode and a voltage applied to the secondary electron multiplier may be set higher than a device-specific set value that is preset according to a mass-to-charge ratio range of ions trapped in the ion trap unit of the ion trap mass spectrometer.

According to the method for analyzing a double-stranded oligonucleotide according to Item 2, the double-stranded oligonucleotide can be analyzed with higher sensitivity.

(Item 3) In the method for analyzing a double-stranded oligonucleotide according to Item 1 or 2, the delay time may be 60 ~ 80 microseconds.

According to the method for analyzing a double-stranded oligonucleotide according to Item 3, the double-stranded oligonucleotide can be analyzed with high sensitivity.

(Item 4) In the method for analyzing a double-stranded oligonucleotide according to any one of Items 1 to 3, the ion trap mass spectrometer may be a digital ion trap mass spectrometer.

According to the method for analyzing a double-stranded oligonucleotide according to Item 4, the double-stranded oligonucleotide can be analyzed with higher sensitivity.

(Item 5) In the method for analyzing a double-stranded oligonucleotide according to any one of Items 1 to 4, an analytical sample containing the sample, a matrix, and ammonium citrate dibasic as a matrix additive may be prepared, and the analytical sample may be subjected to mass spectrometry using the ion trap mass spectrometer.

According to the method for analyzing a double-stranded oligonucleotide according to Item 5, an analytical sample suitable for mass spectrometry using a MALDI ion trap mass spectrometer can be prepared, and a double-stranded oligonucleotide can be analyzed.

(Item 6) In the method for analyzing a double-stranded oligonucleotide according to Item 5, when a matrix solution containing the matrix, the ammonium citrate dibasic, and a solvent is prepared, a concentration of the ammonium citrate dibasic in the matrix solution may be 100 to 200 mM.

(Item 7) In the method for analyzing a double-stranded oligonucleotide according to Item 5 or 6, the matrix may at least comprise 3-hydroxypicolinic acid, or be 2',4',6'-trihydroxyacetophenone monohydrate or 2,4-dihydroxyacetophenone.

(Item 8) In the method for analyzing a double-stranded oligonucleotide according to any one of Items 5 to 7, the solvent may be water or a mixed solvent of water and acetonitrile.

According to the method for analyzing a double-stranded oligonucleotide according to any one of Items 6 to 8, the double-stranded oligonucleotide can be analyzed with higher sensitivity.

(Item 9) In the method for analyzing a double-stranded oligonucleotide according to any one of Items 1 to 8, the double-stranded oligonucleotide may be siRNA.

### REFERENCE SIGNS LIST

- 1: Ion source
- 2: Ion trap
- 3: Detection unit
- 11: Laser irradiation unit
- 12: Sample stage
- 21: Ring electrode
- 22, 23: End cap electrodes
- 31: Conversion dynode
- 32: Secondary electron multiplier

## Claims

1. A method for analyzing a double-stranded oligonucleotide included in a sample, the method using an ion trap mass spectrometer comprising an ion source based on matrix-assisted laser desorption/ionization, an ion trap unit for trapping and ejecting ions generated in the ion source, and a detection unit for detecting the ions ejected from the ion trap unit, the method comprising: introducing the sample into the ion source and irradiating the sample with a laser; trapping the ions generated in the ion source in the ion trap unit; and subsequently ejecting and detecting the ions trapped in the ion trap unit, wherein a delay time, which is a time from irradiating the sample with the laser in the ion source until applying a trapping voltage to trap the ions in the ion trap unit, is set to be longer than a value preset for the ion trap mass spectrometer as a delay time for trapping ions having a mass-to-charge ratio comparable to a mass-to-charge ratio corresponding to a molecular weight of the double-stranded oligonucleotide.

2. The method for analyzing a double-stranded oligonucleotide according to claim 1, wherein the detection unit comprises a conversion dynode and a secondary electron multiplier, and wherein at least one of a voltage applied to the conversion dynode and a voltage applied to the secondary electron multiplier is set higher than a device-specific set value that is preset according to a mass-to-charge ratio range of ions trapped in the ion trap unit of the ion trap mass spectrometer.

3. The method for analyzing a double-stranded oligonucleotide according to claim 1, wherein the delay time is 60 ~ 80 microseconds.

4. The method for analyzing a double-stranded oligonucleotide according to claim 1, wherein the ion trap mass spectrometer is a digital ion trap mass spectrometer.

5. The method for analyzing a double-stranded oligonucleotide according to claim 1, comprising: preparing an analytical sample containing the sample, a matrix, and ammonium citrate dibasic as a matrix additive; and subjecting the analytical sample to mass spectrometry using the ion trap mass spectrometer.

6. The method for analyzing a double-stranded oligonucleotide according to claim 5, wherein when a matrix solution containing the matrix, the ammonium citrate dibasic, and a solvent is prepared, a concentration of the ammonium citrate dibasic in the matrix solution is 100 to 200 mM.

7. The method for analyzing a double-stranded oligonucleotide according to claim 5, wherein the matrix at least comprises 3-hydroxypicolinic acid, or is 2',4',6'-trihydroxyacetophenone monohydrate or 2,4-dihydroxyacetophenone.

8. The method for analyzing a double-stranded oligonucleotide according to claim 5, wherein the solvent is water or a mixed solvent of water and acetonitrile.

9. The method for analyzing a double-stranded oligonucleotide according to claim 1, wherein the double-stranded oligonucleotide is siRNA.
